# EUROPEAN PATENT SPECIFICATION

(11) **EP 1 286 970 B1**
(45) Date of publication and mention of the grant of the patent: **28.04.2010**
(21) Application number: 01965047.2
(22) Date of filing: 07.06.2001
(51) Int. Cl.: C07D 231/44, A01N 43/56

(54) **PROCESSES FOR THE PREPARATION OF PESTICIDAL COMPOUNDS AND NOVEL INTERMEDIATES THEREOF**
HERSTELLUNGSMETHODE FÜR SCHÄDLINGSBEKÄMPFUNGSMITTEL UND ZWISCHENPRODUKTE FÜR IHRE HERSTELLUNG
PROCEDES DE PREPARATION DE COMPOSES PESTICIDES ET LEURS NOUVEAUX INTERMEDIAIRES

(30) Priority: 09.06.2000 US 210803 P; 16.01.2001 EP 01100893
(43) Date of publication of application: 05.03.2003
(73) Proprietor: Merial Limited, Duluth, GA 30096-4640 (US)
(72) Inventor: ROUSSEAU, Jean-François, F-93160 Noisy Le Comte (FR); BUFORN, Albert, F-69008 Lyon (FR)
(74) Representative: Clyde-Watson, Zöe
(86) International application number: PCT/EP2001/007399
(87) International publication number: WO 2001/094316

(56) References cited:
- WO-A-00/35884
- US-A- 4 931 461
- US-A- 5 556 873

## Description

The present invention relates to a process for the preparation of substituted pyrazoles and to their use as pesticidal compound.

Pyrazoles such as 5-Amino-1-aryl-3-cyanopyrazole compounds and derivatives thereof for example Fipronil, form an important class of insecticides. Certain substituted 5-N-alkyl-N-alkoxyacetylamino-1-aryl-3-cyanopyrazole compounds have valuable pesticidal properties as disclosed in WO00/35884 and US Patent No. 5,556,873.

US Patent No. 4931461 discloses substituted 5-methylamino-1-aryl pyrazoles and their use as pest-combating agents. These substituted compounds may be prepared in various ways but in particular it has been found that the compounds may be prepared by reacting the pyrazole with an alkylating agent. This preparation method, whilst being effective, produces by-products that must be isolated from the desired pesticidal compound.

We have found an alternative route to the production of the aforementioned compounds which reduces and substantially eliminates the presence of by-products, thus avoiding the need to purify the final product.

Accordingly, the present invention provides a process for the preparation of a compound of General Formula (I): 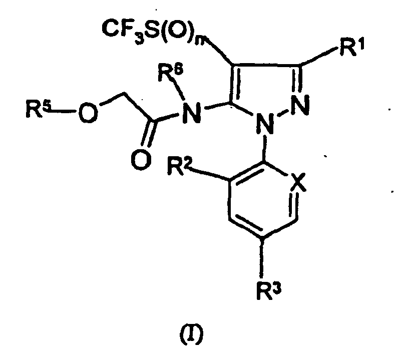 wherein:
R¹ is CN or CSNH₂;
X is N or CR4;
R² and R⁴ are each independently hydrogen or chlorine;
R³ is halogen, haloalkyl, haloalkoxy or -SF₅;
R⁵ and R⁶ are each independently an alkyl group; and
n is 0, 1 or 2;
which process comprises (a) a first step of reacting a compound of formula (II): 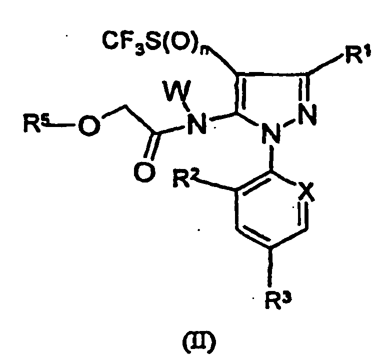 wherein the various symbols are as defined above and W is H, with an inorganic metal salt or an organic base, to afford as an intermediate, a salt of said compound of formula (II); and (b) in a second step, reacting said salt of said compound of formula (II) with an alkylating agent of formula (III):

R⁶-Y (III)

wherein R⁶ is as defined above and Y is a leaving group.

This process provides the advantage over previously known processes in this process is more efficient and provides a more direct route to the final product.

It has been found that prior to reacting compound (II) with the alkylating agent, compound (II) may be reacted initially with an inorganic metal salt or an organic base, thereby forming an intermediate salt which is then reacted with the alkylating agent.

The process of the present invention provides the advantage over the prior art in that there is no by-products produced during the reaction and that if desired, the intermediate compound may be prepared and isolated. The intermediate compound has been found to be stable.

Furthermore, the intermediate compound obtained by the aforementioned process is a novel compound and hereby provides another aspect of the present invention.

The process of the present invention comprises reacting a compound of General Formula (II) with an alkylating agent or optionally first with an inorganic salt or organic base, followed by the alkylating agent. With regard to R³ of Compound II, this group may be halogen, haloalkyl, haloalkoxy or -SF₅. Where R³ is a haloalkyl. Suitable haloalkyls are halomethyls, especially trifluoromethyl. Where R³ is a haloalkoxy, suitable haloalkoxy groups include halomethoxy, in particular trifluoromethoxy. With regard to R⁵, this group is an alkyl group, for example methyl, ethyl or propyl, especially ethyl.

Preferably, the compound of General Formula (II) has the following representations:
R¹ is CN;
X is CR⁴;
R² and R⁴ are each chlorine,
R³ is trifluoromethyl;
R⁵ is ethyl ;
W is H; and
n is 1.

Where the compound of general formula (II) is reacted with the alkylating agent, suitable alkylating agents may be selected from alkyl sulphonates, alkyl halides or alkyl sulphates. The alkyl group may be methyl, ethyl, propyl or isopropyl. Where the alkylating agent is a halide, preferably the agent is chloride, bromide or iodide. Where the alkylating agent is a sulphonate, it is preferred to use di-methyl sulphonate or methyl aryl sulphonate. Where the alkylating agent is a sulphate, the preferred sulphate is dimethyl sulphate. The preferred alkylating agent is methyl bromide, methyl iodide or salts thereof and di-methyl sulphate.

The compound of General Formula (II) is reacted with the alkylating agent in an amount of suitably up to 10 equivalents, preferably from 1 to 20, especially from 5 to 10 equivalents.

The reaction between compound (II) and the alkylating agent may also be carried out in the presence of a base. Suitable bases include alkali metal hydrides, for example sodium hydride; alkali metal carbonates such as potassium carbonate or sodium carbonate or hydrogen carbonates; alkali metal alkoxides for example sodium methoxide; alkali metal hydroxides, for example sodium hydroxide and potassium hydroxide. Alternatively, this reaction may be carried out in the presence of an organic base such as pyridine or tri-ethylamine; or a quaternary ammonium salt such as benzyltriethylammonium halide, for example the chloride or bromide salt or salts of R₄NOH, R₄NOalkyl for example Bu₄NOH. The preferred base is potassium carbonate or potassium hydroxide.

The reaction also may be carried out in the presence of a solvent, preferably a polar organic solvent which may be selected from ethers such as tetrahydrofuran, t-butylmethylether, dioxan, di-isopropyl ether and di-butyl ether; halogenated aromatics or aliphatic hydrocarbons such as dichloromethane, 1,2dichloroethane and monochlorobenzene; polar nitriles and amides such as acetonitrile, N,N-dimethylformamide and N-methyl pyrrolidinone. The preferred solvent is acetonitrile, N,N-dimethylformamide and N-methyl pyrrolidinone. There may also be present a non -polar solvent such as toluene. The solvent is suitably present in excess.

Where the compound of General Formula (II) is initially reacted with an organic base or an inorganic metal salt , the inorganic metal salt may be a Group I or II metal salt selected from cesium, potassium, sodium, calcium and magnesium. Preferably, the metal salt is a potassium or sodium metal salt. The salt may be in the aqueous or solid form and may suitably be a hydroxide, a carbonate or a hydrogen carbonate. The preferred salt for use in the process of the present invention is potassium carbonate or potassium hydroxide. The organic base is suitably an amine, for example triethyl amine, pyridine and the like.

The compound of General Formula (II) is reacted with the metal salt or the organic base in a ratio of at least 1 equivalent, preferably 2 equivalents.

The first step to produce the intermediate compound may be carried out in the presence of a solvent, preferably a polar organic solvent which may be selected from ethers such as tetrahydrofuran, t-butylmethylether, dioxan, di-isopropyl ether and di-butyl ether; halogenated aromatis or aliphatic hydrocarbons such as dichloromethane, 1,2dichloroethane and monochlorobenzene; polar nitriles and amides such as acetonitrile, N,N-dimethylformamide and N-methyl pyrrolidinone or a mixture thereof. The preferred solvent is acetonitrile, N,N-dimethylformamide and N-methyl pyrrolidinone. There may also be present a non -polar solvent such as toluene. The solvent is suitable present in excess.

The intermediate product obtained is a novel product and herewith provides another aspect of the present invention. In particular when the compound of General Formula II is reacted with potassium carbonate to generate the potassium salt or with triethyl amine to produce the amine salt.

The intermediate compound, is then reacted with an alkylating agent of General Formula (III). The alkylating agent may be selected from alkyl sulphonates, alkyl halides or alkyl sulphates. The alkyl group may be methyl, ethyl, propyl or isopropyl. Where the alkylating agent is a halide, preferably the agent is chloride, bromide or iodide. Where the alkylating agent is a sulphonate, it is preferred to use di-methyl sulphonate or methyl aryl sulphonate. Where the alkylating agent is a sulphate, the preferred sulphate is di-methyl sulphate. The preferred alkylating agent is methyl bromide, methyl iodide or salts thereof and di-methyl sulphate.

The ratio of alkylating agent to the intermediate metal salt is suitably up to 10 equivalents, preferably from 1 to 20, especially from 5 to 10 equivalents.

The second step of the process may also be carried out in the presence of a base. Bases suitable for use in this second step include alkali metal hydrides, for example sodium hydride; alkali metal carbonates such as potassium carbonate or sodium carbonate or hydrogen carbonates; alkali metal alkoxides for example sodium methoxide; alkali metal hydroxides, for example sodium hydroxide and potassium hydroxide. Alternatively, the second step may be carried out in the presence of an organic base such as pyridine or tri-ethylamine; or a quaternary ammonium salt such as benzyltriethylammonium halide, for example the chloride or bromide salt or salts of R₄NOH, R₄NOalkyl for example Bu₄NOH. The preferred base is potassium carbonate or potassium hydroxide.

The second step of the reaction also may be carried out in the presence of a solvent, preferably a polar organic solvent which may be selected from ethers such as tetrahydrofuran, t-butylmethylether, dioxan, di-isopropyl ether and di-butyl ether; halogenated aromatics or aliphatic hydrocarbons such as dichloromethane, 1,2dichloroethane and monochlorobenzene; polar nitriles and amides such as acetonitrile, N,N-dimethylformamide and N-methyl pyrrolidinone. The preferred solvent is acetonitrile, N,N-dimethylformamide and N-methyl pyrrolidinone. There may also be present a non -polar solvent such as toluene. The solvent is suitable present in excess.

The process according to the present invention may be carried out at a temperature of from reaction temperature 0°C to 150°C, preferably from 20°C to 90°C and at atmospheric or elevated pressure.

The process of the present invention is particularly preferred for the production of a compound according to General Formula (I) where:
R¹ is CN
X is CR₄
R² and R⁴ are each, chloride
R³ is trifluoromethyl,
R⁵ is ethyl
R⁶ is methyl; and
nis 1

The compounds of formula (II) may be obtained by a process (B), wherein a compound of formula (TV): 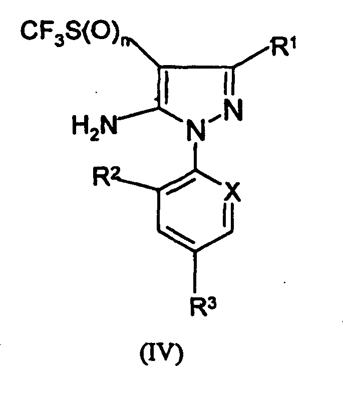 wherein the various symbols are as defined above, is reacted with an acylating agent of formula (V) or formula (VI): 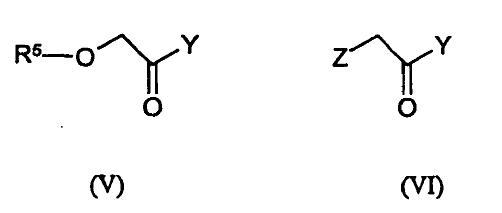 wherein R⁵ is as defined above and Y is a halide, especially chloride or bromide; alkoxy, anhydride, especially halide, e.g. chloride and Z is a halide, for example chloride, bromide and iodide.

The preferred compound of Formula (V) is when R⁵ is ethyl and Y is chloride and for Compound (VI), when Z is chloride and Y is chloride.

The process (B) is preferably carried out in the presence of a solvent, preferably a polar organic solvent which may be selected from ethers such as tetrahydrofuran, t-butylmethylether, dioxan, di-isopropyl ether and di-butyl ether; halogenated aromatic or aliphatic hydrocarbons such as dichloromethane, 1,2dichloroethane and monochlorobenzene; polar nitriles and amides such as acetonitrile, N,N-dimethylformamide and N-methyl pyrolidinone or a mixture thereof. The preferred solvent is acetonitrile, N,N-dimethylformamide and N-methyl pyrolidinone. There may also be present a non -polar solvent such as toluene. The solvent is suitable present in excess.

The process (B) is also preferably carried out in the presence of an organic or inorganic base. Bases suitable for use in this process include alkali metal hydrides, for example sodium hydride; alkali metal carbonates such as potassium carbonate or sodium carbonate or hydrogen carbonates; alkali metal alkoxides for example sodium methoxide; alkali metal hydroxides, for example sodium hydroxide and potassium hydroxide. Alternatively, the reaction may be carried out in the presence of an organic base such as pyridine or tri-ethylamine; or a quaternary ammonium salt such as benzyltriethylammonium halide, for example the chloride or bromide salt or salts of R₄NOH, R₄NOalkyl for example Bu₄NOH. The preferred base is potassium hydroxide, sodium hydroxide and tri-ethylamine. The reaction temperature is generally from minus 20°C to 150°C, preferably from 20°C to 90°C.

In a particular embodiment of the present invention, when compound (VI) is used to produce Compound II, and Z and Y are each chloride, this compound is reacted in the presence of a metal alkoxide, for example sodium ethoxide.

Compounds of formula (III), (IV) and (V) and (VI) are known or may be prepared by known methods.

The intermediate salt of compound (II) may also be obtained directly from the medium reaction of compound (IV) with compound (V) as discussed above. The isolation of this salt may be carried out by the filtration or by the addition of any suitable solvent.

The present invention will now be illustrated by reference to the following examples:

### Example 1

Step 1: Preparation of the potassium salt of 1-(2,6-dichloro-4-trifluoromethylphenyl)-3-cyano-4-trifluoromethylsulfinyl-5-(ethoxyacetamido)pyrazole.

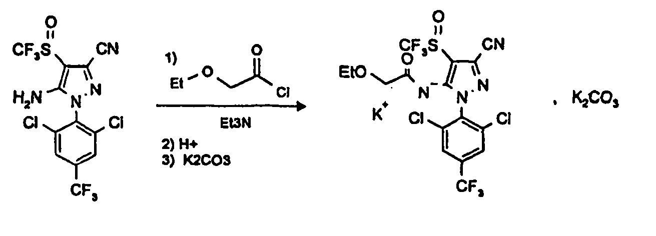

30g of ethoxyacetyl chloride (0.233mol) was added to a mixture of 1-(2,6-dichloro-4-trifluoromethylphenyl)-3-cyano-4-trifluoromethylsulfinyl-5-amino-pyrazole (66g, 0.145mol) and triethylamine (44.5g, 0.435mol) in 100ml of tetrahydrofurane. The reaction mixture was stirred at 30°C during 5h, allowed to cool and 150mL of water and 150mL of CH2Cl2 were added. The pH was reduced to pH 2 with concentrated hydrochloric acid and the product extracted with CH2Cl2. A solution of potassium carbonate (50%) was added and the resulting precipitate concentrated to provide Compound II wherein W is potassium. (yield = 65%, assay = 77%).

Step 2: Preparation of 1-(2,6-dichloro-4-trifluoromethylphenyl)-3-cyano-4-trifluoromethylsulfinyl-5-(ethoxyacetamido- methyl)pyrazole.

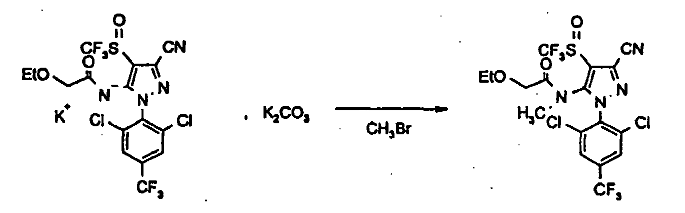

To a suspension of the potassium salt of 1-(2,6-dichloro-4-trifluoromethylphenyl)-3-cyano-4-trifluoromethylsulfinyl-5-e(ethoxyacetamido)pyrazole, prepared in Step 1 above, (18.9g, assay =75.6%, 0.026mole) in 56.8g of acetonitrile, a solution of methyl bromide in acetonitrile (86.5g, conc =28%, 0.255mole) was added. The mixture was stirred during 6 hours at 60°C and then concentrated to dryness. The residue was solubilized in a mixture of toluene (100g) and water (100g). The organic layer was washed with 100g of water and concentrated to a 38% solution, heated to 80°C and product was recrystallized in a 40/60 toluene/n-heptane solution to afford 10.3g of a white solid (yield = 64%, assay =85 %).

### Example 2

Step 1: Preparation of the TEA salt of 1-(2,6-dichloro-4-trifluoromethylphenyl)-3-cyano-4-trifluoromethylsulfinyl-5-(ethoxyacetamido)pyrazole.

3.32g of ethoxyacetyl chloride (0.03mol) was added to a mixture of 1-(2,6-dichloro-4-trifluoromethylphenyl)-3-cyano-4-trifluoromethylsulfinyl-5-amino-pyrazole (8.74g, 0.02mol) and triethylamine (8.4ml, 0.06mol) in 20ml of tetrahydrofuran. The reaction mixture was stirred at 60°C during 1h and 1.1g (0.01mmol) of ethoxyacetyl chloride was added to the medium. After stirring for 30 minutes, the reaction mixture was allowed to cool and 20 ml of water and 20 ml of CH₂Cl₂ were added. The organic layer was washed with 10 ml of water and dried over magnesium sulphate. 12.5 g of Compound II, wherein W is triethylamine, was obtained giving a yield of 90% and an assay of 76%.

Step 2: 0.42 mole of the tri ethyl amine salt of 1-(2,6-dichloro-4-trifluoromethylphenyl)-3-cyano-4-trifluoromethylsulfinyl-5-(ethoxyacetamido)pyrazole, prepared according to step 1 above, was disccolved in 5ml of CH₂Cl₂. The pH was acidified to pH 2 with concentrated hydrochloric acid and the organic layer separated. The organic layer was then treated with a concentrated solution of NaOH (1.5 equivalents) and iodomethane (1.5 equivalents) to provide a yield of 40% of Compound I.

### Example 3

Step 1: 3.1g of Ethoxyacetyl chloride (0.024mol) was added during 2h to a mixture of 1-(2,6-dichloro-4-trifluoromethylphenyl)-3-cyano-4-trifluoromethylsulfinyl-5-amino-pyrazole (10g, 0.022mol) and KOH (3.2g, 0.57mol) in 7g of CH3CN. The reaction mixture was stirred at -5°C during 2h and the resulting mixture filtered : 15g of the wet solid was obtained. After drying 12.2g of compound II, wherein W is potassium, was obtained (yield = 87%, assay = 82%).

Step 2: Preparation of 1-(2,6-dichloro-4-trifluoromethylphenyl)-3-cyano-4-trifluoromethylsulfinyl-5-(ethoxyacetamido- methyl)pyrazole.

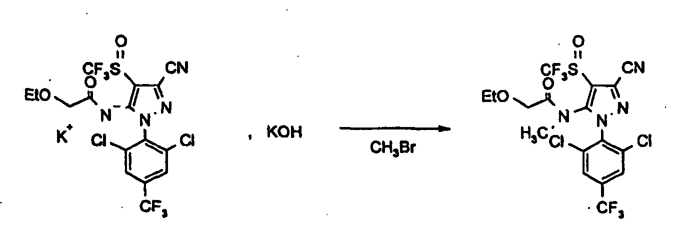

To a suspension of the potassium salt of 1-(2,6-dichloro-4-trifluoromethylphenyl)-3-cyano-4-trifluoromethylsulfinyl-5-(ethoxyacetamido)pyrazole (0.251g, assay =82%, 0.36mmole) in 1.3g of acetonitrile, a solution of methyl bromide in acetonitrile (0.7g, conc =28%, 2.1mole) was added. The mixture was stirred during 6 hours at 60°C in a pressure vessel. Chemical Yield of the final compound is 85%.

### Example 4

1 equivalent of fipronil was reacted with 0.65 equivalents of ethoxyacetylchloride in tetrahydrofuran with 3 equivalents of triethylamine and a trace of 4-dimethylaminopyridine to provide a 75% yield based on the acetylchloride of 3-cyano-1-(2,-6-dichloro-4-trifluoromethylphenyl)-5-ethoxyacetamido-4-trifluoromethylsulfinylpyrazole.

The product was then treated with 1:1 equivalent of dimethyl sulphate and 1:1 equivalent of potassium carbonate in tetrahydrofuran at 25°C for 4 hours to provide 3-cyano-1-(2,-6-dichloro-4-trifluoromethylphenyl)-5-N-ethoxyacetamido-N-methyl-4-trifluoromethylsulfinylpyrazole.

## Claims

1. A process for the preparation of a compound of General Formula (I): 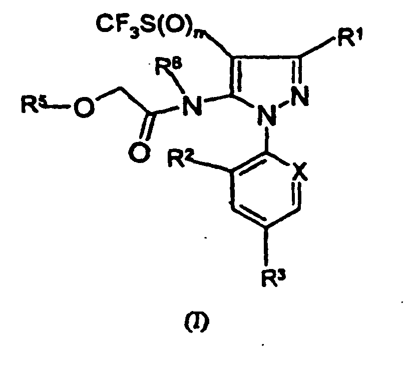 wherein:
R¹ is CN or CSNH₂;
X is N or CR⁴;
R² and R⁴ are each independently hydrogen or chlorine;
R³ is halogen, haloalkyl, haloalkoxy or -SF₅;
R⁵ and R⁶ are each independently an alkyl group; and
n is 0, 1 or 2;
which process comprises (a) a first step of reacting a compound of formula (II): 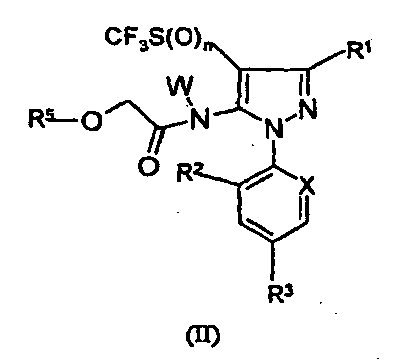
wherein the various symbols are as defined above and W is H, with an inorganic metal salt or an organic base, to afford as an intermediate, a salt of said compound of formula (II); and (b) in a second step, reacting said salt of said compound of formula (II) with an alkylating agent of formula (III) :
R⁶-Y
wherein R⁶ is as defined above and Y is a leaving group.

2. A process as claimed in claim 1 in which the alkylating agent is an alkyl halide, alkyl sulphonate or an alkyl sulphate.

3. A process as claimed in claim 2 in which the alkylating agent is methyl bromide, methyl iodide or dimethyl sulphonate.

4. A process as claimed in any one of the preceding claims carried out in the presence of a solvent.

5. A process as claimed in any one of the preceding claims wherein steps 1 and 2 are carried out in the presence of a base

6. A process as claimed in claim 1 in which inorganic metal salt is a Group I or Group II metal salt selected from cesium, potassium, sodium, magnesium and calcium.

7. A process as claimed in claim 6 in which the inorganic salt is a hydroxide, a carbonate or a hydrogen carbonate.

8. A process as claimed in any one of the preceding claims in which the inorganic salt is potassium carbonate or potassium hydroxide.

9. A process as claimed in any one of the preceding claims in which the organic base is an amine selected from triethyl amine, pyrridine and the like.

10. A process as claimed in any one of the preceding claims in which the compound of General Formula (II) is reacted with the metal salt or the organic base in a ratio of at least one equivalent, preferably 2 equivalents.

11. A process as claimed in any one of the preceding claims in which the compound of General Formula (II) has representations R¹ is CN; X is CR⁴; R² and R⁴ are each chloride, R³ is trifluoromethyl; R⁵ is ethyl, W is an inorganic salt or an organic base; and n is 1.

12. Novel compound as claimed as the intermediate compound as defined in any one of the preceding claims according to General Formula (II) wherein W is an inorganic salt or an organic base.

13. Novel compound as claimed in claim 12 in which W is K⁺.

14. A process for the preparation of a compound of formula (II) as defined in claim 1 which process comprises the reaction of a compound of formula (IV): 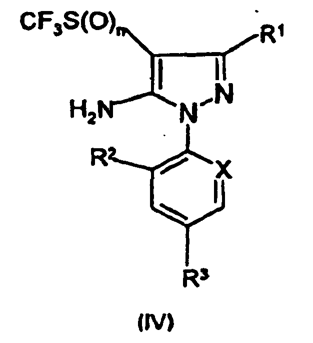 wherein the various symbols are as defined above, with an acylating agent of formula (V) or (VI): 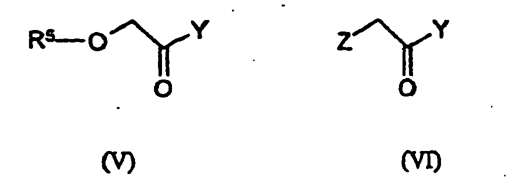 wherein R⁵ and Y are as defined above and Z is a chloride, bromide or iodide.

15. A process as claimed in claim 14 wherein R⁵ and Y of compound (V) are ethyl and chloride respectively.

16. A process as claimed in claim 14 wherein Z and Y of compound (VI) are each chloride.

17. A process as claimed in any one of claims 14 to 16 carried out in the presence of a solvent.

18. A process as claimed in any one of claims 14 to 17 carried out in the presence of an inorganic or organic base.

19. A process for the preparation of novel compounds as defined in claim 12 or 13 which comprises the addition of a solvent to the reaction medium between compound (IV) and compound (V) of the process as claimed claim 14.

## Patentansprüche

1. Ein Verfahren zur Herstellung einer Verbindung der allgemeinen Formel (I): 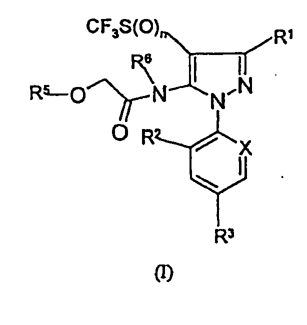 wobei:
R¹ CN oder CSNH₂ ist;
X N oder CR⁴ ist;
R² und R⁴ jeweils unabhängig Wasserstoff oder Chlor sind;
R³ Halogen, Halogenalkyl, Halogenalkoxy oder -SF₅ sind;
R⁵ und R⁶ jeweils unabhängig ein Alkylrest sind; und
n 0, 1 oder 2 ist;
wobei das Verfahren (a) einen ersten Schritt des Umsetzens einer Verbindung der Formel (II): 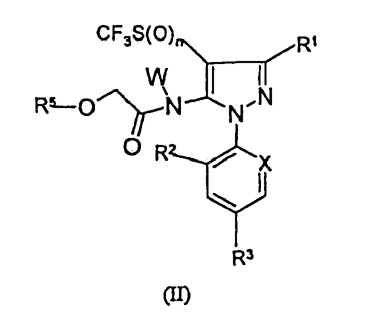
wobei die verschiedenen Symbole wie vorstehend definiert sind und W H ist, mit einem anorganischen Metallsalz oder einer organischen Base, um ein Salz der Verbindung der Formel (II) als ein Zwischenprodukt zu erhalten; und (b), in einen zweiten Schritt, das Umsetzen des Salzes der Verbindung der Formel (II) mit einem Alkylierungsmittel der Formel (III):
R⁶-Y (III)
wobei R⁶ wie vorstehend definiert ist und Y eine Abgangsgruppe ist, umfasst.

2. Ein Verfahren wie in Anspruch 1 beansprucht, wobei das Alkylierungsmittel ein Alkylhalogenid, Alkylsulfonat oder ein Alkylsulfat ist.

3. Ein Verfahren wie in Anspruch 2 beansprucht, wobei das Alkylierungsmittel Methylbromid, Methyliodid oder Dimethylsulfonat ist.

4. Ein Verfahren wie in einem der vorstehenden Ansprüche beansprucht, welches in Gegenwart eines Lösungsmittels durchgeführt wird.

5. Ein Verfahren wie in einem der vorstehenden Ansprüche beansprucht, wobei die Schritte 1 und 2 in Gegenwart einer Base durchgeführt werden.

6. Ein Verfahren wie in Anspruch 1 beansprucht, wobei das anorganische Metallsalz ein Metallsalz der Gruppe I oder Gruppe II, ausgewählt aus Cäsium, Kalium, Natrium, Magnesium und Calcium, ist.

7. Ein Verfahren wie in Anspruch 6 beansprucht, wobei das anorganische Salz ein Hydroxid, ein Carbonat oder ein Hydrogencarbonat ist.

8. Ein Verfahren wie in einem der vorstehenden Ansprüche beansprucht, wobei das anorganische Salz Kaliumcarbonat oder Kaliumhydroxid ist.

9. Ein Verfahren wie in einem der vorstehenden Ansprüche beansprucht, wobei die organische Base ein Amin, ausgewählt aus Triethylamin, Pyrridin und dergleichen, ist.

10. Ein Verfahren wie in einem der vorstehenden Ansprüche beansprucht, wobei die Verbindung der allgemeinen Formel (II) mit dem Metallsalz oder der organischen Base in einem Verhältnis von mindestens 1 Äquivalent, bevorzugt 2 Äquivalenten, umgesetzt wird.

11. Ein Verfahren wie in einem der vorstehenden Ansprüche beansprucht, wobei die Verbindung der allgemeinen Formel (II) die Darstellungen R¹ ist CN; X ist CR⁴; R² und R⁴ sind jeweils Chlorid; R³ ist Trifluormethyl; R⁵ ist Ethyl; W ist ein anorganisches Salz oder eine organische Base; und n ist 1 aufweist.

12. Neue Verbindung wie als die Zwischenverbindung wie in einem der vorstehenden Ansprüche gemäß der allgemeinen Formel (II) definiert beansprucht, wobei W ein anorganisches Salz oder eine organische Base ist.

13. Neue Verbindung wie in Anspruch 12 beansprucht, wobei W K⁺ ist.

14. Ein Verfahren zur Herstellung einer Verbindung der Formel (II) wie in Anspruch 1 definiert, wobei das Verfahren das Umsetzen einer Verbindung der Formel (IV): 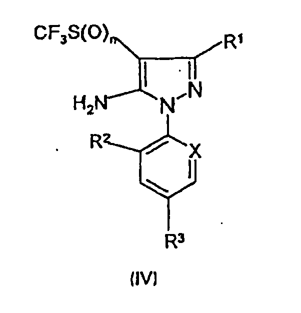 wobei die verschiedenen Symbole wie vorstehend definiert sind, mit einem Acylierungsmittel der Formel (V) oder (VI): 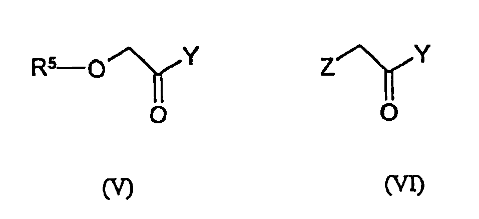 wobei R⁵ und Y wie vorstehend definiert sind und Z ein Chlorid, Bromid oder Iodid ist, umfasst.

15. Ein Verfahren wie in Anspruch 14 beansprucht, wobei R⁵ und Y der Verbindung (V) Ethyl beziehungsweise Chlorid sind.

16. Ein Verfahren wie in Anspruch 14 beansprucht, wobei Z und Y der Verbindung (VI) jeweils Chlorid sind.

17. Ein Verfahren wie in einem der Ansprüche 14 bis 16 beansprucht, durchgeführt in der Gegenwart eines Lösungsmittels.

18. Ein Verfahren wie in einem der Ansprüche 14 bis 17 beansprucht, durchgeführt in der Gegenwart einer anorganischen oder organischen Base.

19. Ein Verfahren zur Herstellung von neuen Verbindungen wie in Anspruch 12 oder 13 definiert, welches das Hinzufügen eines Lösungsmittels zum Reaktionsmedium zwischen Verbindung (IV) und Verbindung (V) des Verfahrens, wie in Anspruch 14 beansprucht, umfasst.

## Revendications

1. Procédé de préparation d'un composé de formule générale (I) : 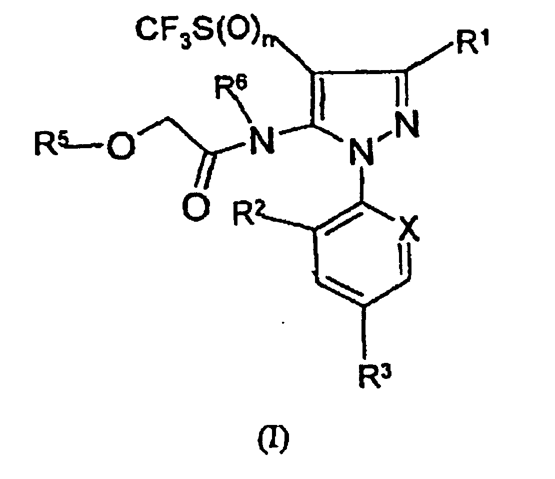 dans laquelle :
R¹ est CN ou CSNH₂ ;
X est N ou CR⁴ ;
R² et R⁴ sont chacun indépendamment un atome d'hydrogène ou de chlore ;
R³ est un halogène, un haloalkyle, haloalcoxy ou -SF₅ ;
R⁵ et R⁶ sont chacun indépendamment un groupe alkyle ; et
n vaut 0, 1 ou 2 ;
le procédé comprenant (a) une première étape consistant à faire réagir un composé de formule (II) : 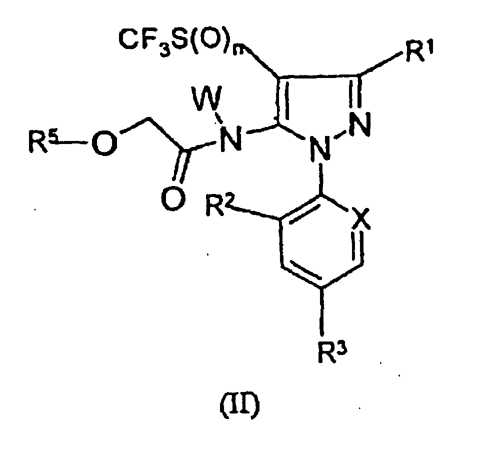
dans laquelle les différents symboles sont tels que définis ci-dessus et W est H, avec un sel de métal inorganique ou une base organique, pour donner en tant qu'intermédiaire, un sel dudit composé de formule (II) ; et (b) une deuxième étape consistant à faire réagir ledit sel dudit composé de formule (II) avec un agent d'alkylation de formule (III) :
R⁶-Y (III)
dans laquelle R⁶ est tel que défini ci-dessus et Y est un groupe partant.

2. Procédé selon la revendication 1, dans lequel l'agent d'alkylation est un halogénure d'alkyle, sulfonate d'alkyle ou un sulfate d'alkyle.

3. Procédé selon la revendication 2, dans lequel l'agent d'alkylation est le bromure de méthyle, l'iodure de méthyle ou le sulfonate de diméthyle.

4. Procédé selon l'une quelconque des revendications précédentes, réalisé en présence d'un solvant.

5. Procédé selon l'une quelconque des revendications précédentes, dans lequel les étapes (a) et (b) sont réalisées en présence d'une base.

6. Procédé selon la revendication 1, dans lequel le sel de métal inorganique est un sel d'un métal du groupe I ou du groupe II choisi parmi le césium, le potassium, le sodium, le magnésium et le calcium.

7. Procédé selon la revendication 6, dans lequel le sel inorganique est un hydroxyde, un carbonate ou un hydrogénocarbonate.

8. Procédé selon l'une quelconque des revendications précédentes, dans lequel le sel inorganique est le carbonate de potassium ou l'hydroxyde de potassium.

9. Procédé selon l'une quelconque des revendications précédentes, dans lequel la base organique est une amine choisie parmi la triéthylamine, la pyridine et équivalents.

10. Procédé selon l'une quelconque des revendications précédentes, dans lequel le composé de formule générale (II) réagit avec le sel de métal ou la base organique en un rapport d'au moins un équivalent, de préférence de 2 équivalents.

11. Procédé selon l'une quelconque des revendications précédentes, dans lequel dans le composé de formule générale (II), R¹ est CN ; X est CR⁴ ; R² et R⁴ sont chacun un chlorure, R³ est un trifluorométhyle ; R⁵ est un éthyle ; W est un sel inorganique ou une base organique ; et n vaut 1.

12. Nouveau composé tel que revendiqué en tant que composé intermédiaire selon l'une quelconque des revendications précédentes de formule générale (II) dans laquelle W est un sel inorganique ou une base organique.

13. Nouveau composé selon la revendication 12, dans lequel W est K⁺.

14. Procédé de préparation d'un composé de formule (II) tel que défini dans la revendication 1, le procédé comprenant la réaction d'un composé de formule (IV) : 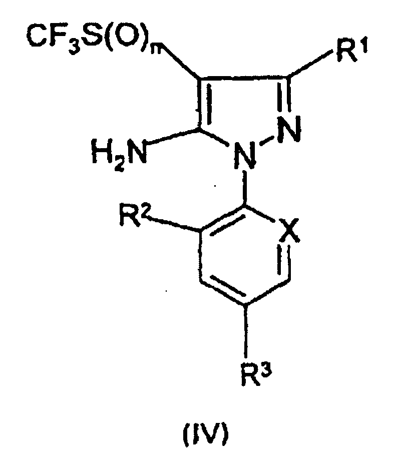 dans laquelle les différents symboles sont tels que définis ci-dessus, avec un agent d'acylation de formule (V) ou (VI) : 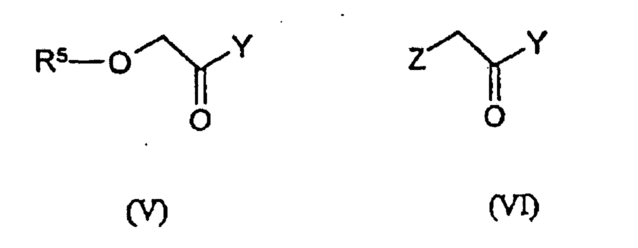 dans lesquelles R⁵ et Y sont tels que définis ci-dessus et Z est un chlorure, un bromure ou un iodure.

15. Procédé selon la revendication 14, dans lequel R⁵ et Y du composé (V) sont un groupe éthyle et un chlorure, respectivement.

16. Procédé selon la revendication 14, dans lequel Z et Y du composé (VI) sont chacun un chlorure.

17. Procédé selon l'une quelconque des revendications 14 à 16, réalisé en présence d'un solvant.

18. Procédé selon l'une quelconque des revendications 14 à 17, réalisé en présence d'une base inorganique ou organique.

19. Procédé de préparation de nouveaux composés selon la revendication 12 ou la revendication 13, qui comprend l'addition d'un solvant au milieu de réaction entre le composé (IV) et le composé (V) du procédé selon la revendication 14.
